# EUROPEAN PATENT SPECIFICATION

(11) **EP 0 862 428 B1**
(45) Date of publication and mention of the grant of the patent: **02.08.2006**
(21) Application number: 96938209.2
(22) Date of filing: 08.11.1996
(51) Int. Cl.: A61K 31/365

(54) **GINKGOLIDES FOR INHIBITION OF MEMBRANE EXPRESSION**
GINKOLIDE ZUR HEMMUNG DER MEMBRANEXPRESSION
GINKGOLIDES DESTINES A INHIBER L'EXPRESSION MEMBRANAIRE

(30) Priority: 09.11.1995 US 7337 P; 20.12.1995 US 575902
(43) Date of publication of application: 09.09.1998
(73) Proprietor: SOCIETE DE CONSEILS DE RECHERCHES ET D'APPLICATIONS SCIENTIFIQUES S.A. (S.C.R.A.S.), 75016 Paris (FR)
(72) Inventor: DRIEU, Katy, F-75116 Paris (FR)
(74) Representative: Owen, Deborah Jane
(86) International application number: PCT/EP1996/005005
(87) International publication number: WO 1997/017068

(56) References cited:
- GEN. PHARMAC., vol. 25, no. 5, 1994, pages 1009-1016, XP002026672 RAPIN J.R. ET AL: "Demonstration of the "antistress" activity of an extract of Ginkgo biloba (EGb 761) using a discrimination learning task" cited in the application
- PHYTOTHER. RES. (UNITED KONGDOM), vol. 7, no. 2, 1993, pages 139-145, XP002026673 PETKOV V.D ET AL: "Effects of Standardized Extracts GK501, from Ginkgo biloba L., G115, from Panax ginseng C.A. Meyer, and their combination, Gincosan (PHL-00701), on the brain levels of biogenic monoamines and on the serum content of prolactin, growth hormone and ACTH "
- "Rote Liste" 1994 , EDITIO CANTOR , AULENDORF/WÜRTT. XP002026674 no. 36001 - 36012

## Description

### Field of the Invention

The invention relates to the inhibition of membrane expression of benzodiazepine receptors and in particular to the use of ginkgolides for the manufacture of medicaments for such membrane expression inhibition.

### Background of the Invention

The steroid glucocorticoid is produced by adrenal fasciculata-reticula cells in the adrenal glands, and are secreted in response to an increase in the level of plasma adrenocorticotrophic hormone (ACTH). Glucocorticoids are involved in carbohydrate, protein, and fat metabolism, have been shown to have antiinflammatory properties, and are hypersecreted during stress. In excess, glucocorticoids have been shown to damage hippocampus, a structure in the limbic system of the brain that is critical to cognitive functions such as learning and memory. See, e.g., Sapolsky, R.M., Ann. N.Y. Acad. Sci. 746:294 (1994); and McEwen, B.S.; Ann. N.Y. Acad. Sci. 746:134 (1994). Furthermore, glucocorticoid neurotoxicity and neuroendangerment has been shown to be critical in neural development and aging as well as in neurological diseases related to hippocampal damage. See, e.g., deKloet, E.R., et al., Ann. N.Y. Acad. Sci. 746:8 (1994)

Studies have been conducted to examine the beneficial effects of extract of the leaves of the gymnosphermus tree ginkgo biloba (e.g., EGb 761) on "antistress" activity by lowering corticosterine levels in stressed rat models. See, Rapin, et al., Gen. Pharmac. 25(5) :1009 (1994). EGb 761 had previously been shown to have activity in the cardiovascular system (e.g., reduction of platelet adhesion and thrombi growth), central nervous system (e.g., neuroprotective activity), and neurosensory system (e.g., retinal protection). See, e.g., DeFeudis, et al., Ginkgo Biloba Extract (EGb 761): Pharmaceutical Activities and Clinical Applications (Elsevier, Paris, 1991).

It has now been found that ginkgolides are effective at inhibiting membrane expression of benzodiazepine receptors, eg. adrenal benzodiazepine receptors, and that, having this effect, they can be used to inhibit glucocorticoid release.

### Summary of the Invention

Thus viewed from one aspect the invention provides the use of ginkgolide A or ginkgolide B, or a pharmaceutically acceptable salt thereof, for the manufacture of a medicament for use in the treatment of Cushings syndrome, stress-induced hypercorticolism or to enhance brain function or inhibit brain ageing wherein said ginkgolide is purified from a ginkgo biloba tree or is produced synthetically.

Ginkgolides may also be used for the manufacture of a medicament for use as an inhibitor of membrane expression of a benzodiazepine receptor, eg. to inhibit glucocorticoid release in a patient. Alternatively an inhibitor of membrane expression of an adrenal benzodiazepine receptor, eg. a ginkgolide, may be used for the manufacture of a medicament for inhibiting glucocorticoid release, eg. to combat (ie. prevent or treat) conditions associated with excess glucocorticoid production.

An exemplary pharmaceutical composition for use in the treatment of Cushings syndrome, stress-induced hypercorticolism or to enhance brain function or inhibit brain ageing, comprises ginkgolide A or ginkgolide B, or a pharmaceutically acceptable salt thereof together with at least one pharmaceutically acceptable carrier or excipient.

An exemplary pharmaceutical composition for use as an inhibitor of membrane expression of benzodiazepine receptors (or for combatting conditions associated with excess glucocorticoid production, etc.), comprises a physiologically tolerable ginkgolide together with at least one pharmaceutically acceptable carrier or excipient.

An exemplary pharmaceutical composition for use as an inhibitor of glucocorticoid release, said composition comprising an inhibitor of membrane expression of an adrenal benzodiazepine receptor (eg. a ginkgolide) together with at least one pharmaceutically acceptable carrier or excipient.

The invention can also be used in a method of treating Cushings syndrome, stress-induced hypercorticolism or to enhance brain function or inhibit brain ageing, said method comprising administering to said patient an effective amount of ginkgolide A or ginkgolide B or a pharmaceutically acceptable salt thereof.

Other exemplary uses are in inhibiting the membrane expression of a benzodiazepine receptor in a patient (eg. a human or non-human, preferably a mammal), said method comprising administering to said patient an effective amount of a ginkgolide, or in a method of inhibiting the release of a glucocorticoid in a patient, said method comprising administering to said patient an effective amount of a compound, eg. a ginkgolide, capable of inhibiting the membrane expression of an adrenal benzodiazepine receptor.

Reduction in excess glucocorticoid levels can, as discussed below, result in enhancement of ACTH levels with various consequent beneficial effects.

Thus a still further exemplary use of the invention is the use of a ginkgolide (or other inhibitor of membrane expression of an adrenal benzodiazepine receptor) for the manufacture of a medicament for enhancing ACTH levels.

Thus one aspect of the invention involves inhibiting the membrane expression of a benzodiazepine receptor. This involves administering to a patient an effective amount of ginkgolide A or B. The benzodiazepine receptor may be a peripheral-type benzodiazepine receptor (PBR), e.g. found on the adrenal, intestine, kidney, brain, liver, and testis. In one embodiment, the membrane is on adrenal mitochondria. In a further exemplary method, this method comprises administering an effective amount of an extract from ginkgo biloba. In another further embodiment this method comprises administering an effective amount of a pharmaceutical composition which contains ginkgolide A or ginkgolide B and a pharmaceutically acceptable carrier.

However another aspect of the invention involves inhibiting the release of a glucocorticoid (such as cortisol) in a patient. This involves the step of administering to the patient an effective amount of a compound capable of inhibiting the membrane expression of an adrenal benzodiazepine receptor. In one embodiment, this method comprises administering to said patient an effective amount of ginkgolide A or B. In a further exemplary method, this method comprises administering an effective amount of an extract from ginkgo biloba. In another further embodiment, this method comprises the step of administering to the patient an effective amount of a pharmaceutical composition containing ginkgolide A or B and a pharmaceutically acceptable carrier.

An effective amount depends upon the condition being treated, the route of administration chosen, and the specific activity of the compound used, and ultimately will be decided by the attending physician or veterinarian. The compound may be administered in an amount of 0.1 to 20 mg/kg body weight of the patient (e.g., 0.5 to 4 mg/kg body weight of the patient).

The pharmaceutical composition described above contains (1) one or more of the ginkgolides to be described below, (2) one or more pharmaceutically acceptable carriers, and, optionally, (3) one or more other ingredients such as another bioactive compound or a stabilizing agent. Any extract from the ginkgo biloba tree is not considered as such a pharmaceutical composition. The carrier must be "pharmaceutically acceptable" in the sense of being compatible with the ginkgolide(s) of the composition and not deleterious to the subject to be treated.

The compositions may conveniently be presented in unit dosage form and may be prepared by any of the methods well known in the art of pharmacy. All methods include the step of bringing the compound(s) (e.g., ginkgolide) into association with a carrier which may contain one or more accessory ingredients. In general, the compositions for tablets (e.g., for oral administration) or powders are prepared by uniformly and intimately blending the compound(s) with finely divided solid carriers, and then, if necessary as in the case of tablets, forming the product into the desired shape and size.

Compositions suitable for parenteral administration (e.g., subcutaneous, intravenous, or intermuscular), on the other hand, conveniently comprise sterile aqueous solutions of the compound(s). Preferably, the solutions are isotonic with the blood of the subject to be treated. Such compositions may be conveniently prepared by dissolving solid compound(s) in water or saline to produce an aqueous solution, and rendering said solution sterile. The composition may be presented in unit or multi-dose containers, for example, sealed ampoules or vials.

The extracts of the ginkgo biloba tree may be prepared by standard extraction techniques. See, e.g., the book, *"Ginkgolides - Chemistry,* Biology, Pharmacology and Clinical Perspectives", edited by P. Braquet (J.R. Prous, Science Publishers, Barcelona, Spain 1988).

Other features and advantages of the present invention will be apparent from the detailed description of the invention and from the claims.

### Detailed Description of the Invention

It is believed that one skilled in the art can, based on the description herein, utilize the present invention to its fullest extent. The following specific embodiments are, therefore, to be construed as merely illustrative, and not limitative of the remainder of the disclosure in any way whatsoever.

Unless defined otherwise, all technical and scientific terms used herein have the same meaning as commonly understood by one of ordinary skill in the art to which this invention belongs. Also, all publications cited herein are incorporated by reference.

### Ginkgolides

The term "ginkgolide" are used herein to include all the naturally occurring ginkgolides which are derived from the ginkgo biloba tree as well as synthetically produced ginkgolides and pharmaceutically active derivatives and salts thereof. Thus, it includes (1) the various ginkgolides disclosed in the books "Ginkgolides - Chemistry, Biology, Pharmacology and Clinical Perspectives", edited by P. Braquet (J.R. Prous, Science Publishers, Barcelona, Spain 1988); F.V. DeFeudis, Ginkgo Biloba Extract (EGb 761), Pharmacological Activities and Chemical Applications (Elsevier, Paris, France 1991); Rokan Ginkgo Biloba - Recent Results in Pharmacology and Clinic, edited by E.W. Feufgeld (Springer-Verlag, Berlin, Germany 1988) and in U.S. Patent Nos. 4,734,280 and 5,002,965; and (2) non-toxic, pharmaceutically active derivatives thereof such as 2,3-dehydro, 1-methoxy, and 1-ethoxy derivatives of ginkgolide B, tetrahydro ginkgolide derivatives, acetyl ginkgolide derivatives, and alkyl ester of ginkgolide, e.g., the monoacetate ginkgolide derivatives described in Okabe, et al., J. Chem. Soc.(C) pp. 2201-2206 (1967); and Corey, et al., J. Amer. Chem. Soc. 110:649 (1988).

As described in the book "Ginkgolides - Chemistry, Biology, Pharmacology and Clinical Perspectives", pp. 27-42, edited by P. Braquet (J.R. Prous, Science Publishers, Barcelona, Spain 1988), ginkgolides may be extracted and purified from the leaves of the ginkgo biloba tree. See, e.g., Okabe, J. Chem. Soc. (C) pp. 2201 (1967); and Nakanishi, Pure & Applied Chem. 14:89 (1967). Ginkgolides and ginkgolide derivatives have also been chemically synthesized. See, e.g., Corey, et al., J. Amer. Chem. Soc. 110:649 (1988). Furthermore, ginkgolides are available from various commercial sources such as Sigma Chemical (St. Louis, Missouri, USA) .

Structurally, ginkgolides are twenty carbon molecules with 6 five-membered rings joined together to form a constrained structure which incorporates a t-butyl group. Of the 6 rings, 3 are lactone rings, 2 are carboxylic rings joined by a single carbon to form a spiro-[4,4]nonane ring system, and 1 tetrahydrofuran ring. Examples of ginkgolides are depicted by the following formula: 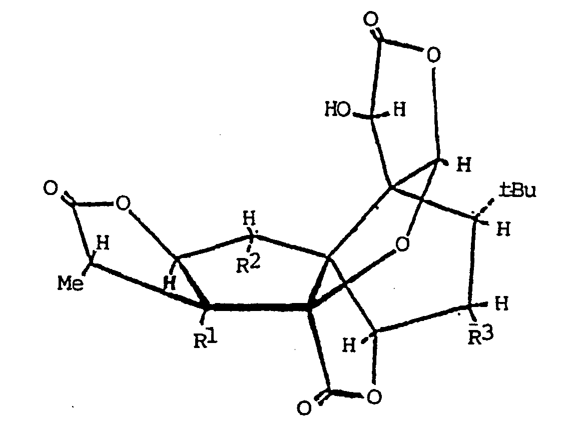 wherein each of R¹, R², and R³, independently, is H, OH, or C₁-C₆ alkoxy, or a pharmaceutically acceptable salt thereof. Examples of ginkgolides include ginkgolide A (R¹ = OH, R² = H, R³ = H), ginkgolide B (R¹ = OH, R² = OH, R³ = H), ginkgolide C (R¹ = OH, R² = OH, R³ = OH) , ginkgolide J (R¹ = OH, R² = H, R³ = OH), and ginkgolide M (R¹ = H, R² = OH, R³ - OH) or the synthetic analogs where R² is C₁-C₆ alkoxy, e.g., 1-methoxy or 1-ethoxy derivatives of ginkgolide B. The term "ginkgolide" also includes all pharmaceutically acceptable salts of ginkgolides, such as sodium, potassium, and magnesium salts thereof. Examples of a ginkgolide to be used to practice the method of this invention has the above formula, in which each, R¹ and R³, independently, is H or OH, and R² is H, OH, or C₁-C₆ alkoxy (such as ginkgolides A, B, C, J, and M); or a pharmaceutically acceptable salt thereof.

### Benzodiazepine Radioligand Binding Assay

The ginkgo biloba extract EGb761, ginkgolide A, and ginkgolide B (Institut Henri Beaufour-IPSEN, Paris, France) were tested for their ability to decrease the number of binding sites for the peripheral benzodiazepine receptor ligand PK 11195, which binds to an 18 Kd peripheral benzodiazepine receptor protein, in adrenal mitochondria. See, Garnier, et al., Endocrinology 132:444 (1993). Mitochondria were prepared as described in Krueger, et al., J. Biol. Chem. 265:15015 (1990). Mitochondria (50 mg of protein) were resuspended in phosphate buffered saline (PBS) and [³H]PK 11195 (New England Nuclear, Wilmington, Delaware, USA). Binding studies were performed at 4°C in a final incubation volume of 0.3 ml, using radioligand in the concentration range of 0.019-20.00 nM and 200 fold excess of unlabeled PK 11195 (Research Biochemicals, Natick, Massachusetts, USA), as described in Garnier, et al., Endocrinology 132:444 (1993) and Garnier, et al., Mol. Pharm. 45:201 (1994). After 120 min. incubation time, the assay was stopped by filtration through Whatman GF/C filters and washed with 15 ml ice-cold PBS. Radioactivity trapped on the filters were determined by liquid scintillation counting at 50% counting efficiency. The dissociation constant (Kd) and the number of binding sites (Bmax) were determined by Standard plot analysis of the data using the ligand™ program (Kell, V.4.0, Biosoft, Inc.). See Munson, et al., Anal. Biochem. 107:220 (1980). The results are shown below in Table I.

**TABLE I**

| | Kd (nM) | Bmax (pmol/mg) |
|---|---|---|
| Control | 1.7 | 11.2 |
| EGb761 | 1.2 | 7.1 |
| Ginkgolide A | 1.3 | 5.6 |
| Ginkgolide B | 1.5 | 3.1 |

Thus, EGb761 decreased the expression of the 18 Kd peripheral benzodiazepine receptor protein by 40%, while ginkgolide A and ginkgolide B reduced the expression by 50% and 73%, respectively.

This finding was verified by immunocytochemical studies using antisera specific for the 18 Kd peripheral benzodiazepine receptor protein. See Oke, et al., Mol. Cell. Endocrinol. 87:R1 (1992) and Garnier, et al., Endocrinology 132:444 (1993). A dramatic decrease in the protein expression was observed after treatment with EGb761, ginkgolide A, and ginkgolide B.

### Immunoblot Analysis of Benzodiazepine Receptor

The ginkgolide induced decrease in the 18 Kd peripheral benzodiazepine receptor protein was also confirmed by immunoblot analysis of mitochondrial extracts obtained from control and treated animals. Adrenal mitochondrial proteins were fractioned by one dimension SDSPAGE and electro-transferred onto nitrocellulose as described in Oke, et al., Mol. Cell. Endocrinol. 87:R1 (1992) and Garnier, et al., Endocrinology 132:444 (1993). The nitrocellulose was subjected to immunoblot analysis using anti-peripheral benzodiazepine receptor antibody and goat IgG-horseradish peroxidase with 4-chloro-1-napthol as color reagent and hydrogen peroxide as substrate. Densiometric analysis of the immunoreactivity protein bonds was performed using Sigmagel™software (Jandel Scientific, San Rafael, California, USA). The densiometric analysis of the immunoreactivity found a 60% decrease of the 18 Kd peripheral benzodiazepine receptor protein by ginkgolide B.

### mRNA Expression of Benzodiazepine Receptor

The ginkgolide induced decrease in mRNA expression of the benzodiazepine receptor was also confirmed. Total cellular RNA from adrenal tissue was isolated by the acid guanidinium thiocyanate-phenol-chloroform extraction method (Chomczynski, et al., Anal. Biochem. 162:156-159 (1987)) using the RNAzol B reagent (Tel-Test Inc., Friendswood, Texas, USA). RNA electrophoresis transfer, probe labelling, and membrane hybridization were performed as previously described in Dym, et al., Endocrinology 128:1167-1176 (1991). RNA was size-fractionated by electrophoresis and transferred to derivatized nylon membranes (Nytran Plus, Schleicher & Schuell, Keene, New Hampshire, USA). The blots were then hybridized against the [³²P]cDNA probe for PBR labelled by the random priming technique. The 781 base-pair probe for PBR mRNA used was prepared as previously described in Garnier, et al., Endocrinology 132:444-458 (1993). Screen enhanced autoradiography was performed by exposing Kodak X-OMAT AR films to the blots at -80°C for 48 hours. Densiometric analysis of the spots was performed as described above. Both EGb761 and ginkgolide B treatment was found to reduce peripheral benzodiazepine receptor mRNA expression by 50% and 85%, respectively.

### Assay for Determining the Inhibition of Glucocorticoids

Adult Sprague-Dawley rats (approximately 300 g; Charles River Laboratories, Wilmington, Massachusetts, USA) were treated once daily for eight days with either ginkgolide A, ginkgolide B, or a saline control. Ginkgolide A and ginkgolide B were injected as an aqueous solution intraperitoneally at a 2 mg/kg. The results shown in Table II are the means of between two to four independent experiments. In each experiment, at least six rats per treatment group were used. After eight days of treatment, the rats were sacrificed.

The level of steroids in the rats was measured by radioimmunoassay from organic extracts of the collected serum. The levels of corticosterone (a glucocorticoid in rats) and testosterone were measured by radioimmunoassay using antibodies from Endocrine Sciences (Tarlana, California, USA) under conditions described by the supplier. The level of plasma ACTH was measured by radioimmunoassay using the method of Crousos, et al., New Engl. J. Med. 310:622 (1984). The level of aldosterone was measured by radioimmunoassay using a kit from Diagnostics Products Corp. (Los Angeles, California, USA). The mean steroid levels for each of the four treatment groups are reported in Table II.

**TABLE II**

| TREATMENT | CORTICOSTERONE ng/ml | ACTH pg/ml | ALDOSTERONE pg/ml | TESTOSTERONE ng/ml |
|---|---|---|---|---|
| Control | 161 | 28.0 | 685 | 4.50 |
| Ginkgolide A | 66 | 103 | 638 | 4.75 |
| Ginkgolide B | 75 | 71.4 | 883 | 4.50 |

Ginkgolide A and ginkgolide B were all found to decrease the level of corticosterone in the rats. Because glucocorticoid secretion induced by the pituitary ACTH is modulated by a negative feedback system on the hypothalamus, the decrease in corticosteroid levels in the rats as a result of the administration of ginkgolide A and ginkgolide B will induce a corresponding increase in pituitary ACTH release and, consequently, plasma ACTH levels.

As shown in Table II, treatment with either ginkgolide A or ginkgolide B was found unexpectedly to cause the rats to naturally respond and increase ACTH release. Furthermore, serum levels of aldosterone (secreted by the adrenal cortex) and testosterone (secreted by the testes) were unaffected by the treatment of ginkgolide A and ginkgolide B, indicating that ginkgolides specifically affect the adrenal fasciculata-reticular cells of the adrenal gland.

### Use

By inhibiting the release of glucocorticoids from the adrenal glands, ginkgolides can be used to treat disorders in patients that are secreting a high level of one or more glucocorticoids. Examples of such patients include those suffering from Cushings syndrome and those with stress-induced hypercorticolism. As discussed above, the levels of ACTH are naturally elevated in response to the suppression of glucocorticoid release upon administration of a ginkgolide. Elevated levels of ACTH or ACTH analogs have been shown to inhibit brain aging (e.g., inhibit neurological loss and improve learning). See, e.g., Laudfield, et al., Science, 214:581 (1981). Thus, ginkgolides enhance brain function by both inhibiting glucocorticoid and maintaining normal ACTH release.

## Claims

1. Use of ginkgolide A or ginkgolide B, or a pharmaceutically acceptable salt thereof, for the manufacture of a medicament for use in the treatment of cushings syndrome, stress-induced hypercorticolism or to enhance brain function or inhibit brain agein wherein said ginkgolide is purified from a ginkgo biloba tree or is produced synthetically.

2. The use as claimed in claim 1 for the manufacture of a medicament for use in the treatment of Cushings syndrome or stress-induced hypercorticolism.

3. Use as claimed in claim 1 or claim 2, wherein said ginkgolide inhibits membrane expression of a benzodiazepine receptor.

4. Use as claimed in claim 3, wherein said benzodiazepine receptor is the peripheral-type benzodiazepine receptor.

5. Use as claimed in claim 3 or claim 4, wherein said membrane is on adrenal mitochondria.

6. Use as claimed in any one of claims 1 to 5, wherein administration of said medicament is effected parenterally or orally.

## Patentansprüche

1. Verwendung von Ginkgolid A oder Ginkgolid B oder eines pharmazeutisch akzeptablen Salzes davon zur Herstellung eines Medikaments zur Verwendung bei der Behandlung des Cushing-Syndroms, von Stress-induziertem Hyperkortisonismus oder zur Steigerung der Hirnfunktion oder zur Verzögerung der Hirnalterung, wobei das Ginkgolid aus einem *Ginkgo* biloba-Baum aufgereinigt oder synthetisch hergestellt ist.

2. Verwendung nach Anspruch 1 zur Herstellung eines Medikaments zur Verwendung bei der Behandlung des Cushing-Syndroms oder von Stress-induziertem Hyperkortisonismus.

3. Verwendung nach Anspruch 1 oder Anspruch 2, wobei das Ginkgolid die Membranexpression eines Benzodiazepin-Rezeptors hemmt.

4. Verwendung nach Anspruch 3, wobei der Benzodiazepin-Rezeptor der Benzodiazepin-Rezeptor vom peripheren Typ ist.

5. Verwendung nach Anspruch 3 oder Anspruch 4, wobei die Membran die Membran auf Mitochondrien der Nebenniere ist.

6. Verwendung nach einem der Ansprüche 1 bis 5, wobei die Verabreichung des Medikaments parenteral oder oral erfolgt.

## Revendications

1. Utilisation de ginkgolide A ou ginkgolide B, ou d'un sel de ceux-ci acceptable d'un point de vue pharmaceutique, pour la production d'un médicament destiné à être utilisé pour le traitement du syndrome de Cushing, de l'hypercorticolisme induit par le stress, ou pour favoriser la fonction cérébrale ou inhiber le vieillissement cérébral, dans laquelle ledit ginkgolide est purifié à partir d'un arbre ginkgo biloba ou est produit de manière synthétique.

2. Utilisation selon la revendication 1 pour la production d'un médicament destiné à être utilisé pour le traitement du syndrome de Cushing ou de l'hypercorticolisme induit par le stress.

3. Utilisation selon la revendication 1 ou selon la revendication 2, dans laquelle ledit ginkgolide inhibe l'expression membranaire d'un récepteur de benzodiazépine.

4. Utilisation selon la revendication 3, dans laquelle ledit récepteur de benzodiazépine est le récepteur de benzodiazépine de type périphérique.

5. Utilisation selon la revendication 3 ou selon la revendication 4, dans laquelle ladite membrane est sur une mitochondrie surrénale.

6. Utilisation selon l'une quelconque des revendications 1 à 5, dans laquelle l'administration dudit médicament est réalisée de manière parentérale ou orale.
